# EUROPEAN PATENT SPECIFICATION

(11) **EP 3 939 455 B1**
(45) Date of publication and mention of the grant of the patent: **22.03.2023**
(21) Application number: 21185760.2
(22) Date of filing: 15.07.2021
(51) Int. Cl.: A41D 19/00, A61B 42/10, B32B 25/14, B29D 99/00

(54) **DETACHABLE BI-LAYERED GLOVE AND METHOD OF MANUFACTURING THEREOF**
ABNEHMBARER DOPPELLAGENHANDSCHUH UND VERFAHREN ZU SEINER HERSTELLUNG
GANT BI-COUCHE AMOVIBLE ET SON PROCÉDÉ DE FABRICATION

(30) Priority: 15.07.2020 MY PI2020003651
(43) Date of publication of application: 19.01.2022
(73) Proprietor: TOP GLOVE INTERNATIONAL SDN. BHD., 41050 Klang Selangor (MY)
(72) Inventor: WONG, Chong Ban, 41050 KLANG, SELANGOR (MY); LING, Siew Szen, 41050 KLANG, SELANGOR (MY); LOW, Meng Lai, 41050 KLANG, SELANGOR (MY); WONG, Sonia How Ming, 41050 KLANG, SELANGOR (MY)
(74) Representative: Comoglio, Elena

(56) References cited:
- US-A- 4 696 065
- US-A1- 2014 289 931

## Description

### FIELD OF THE INVENTION

The present invention relates to a detachable bi-layered glove and method of manufacturing thereof, which provide users the convenience of using same piece of glove for twice without the risk of contamination and the hassle of doffing and donning for twice.

### BACKGROUND OF THE INVENTION

In numerous industries such as healthcare, pharmaceutical and food, double gloving has been a common practice. Simply, a double layered protection has become a preferable option when users deal with high risk chemicals. Double gloving instead of single gloving is advisable since it provides extra protection and it reduces the risk of contamination. Further, single gloving has a major drawback, whereby the users tend to doff the used single-layered glove and don a fresh piece of the glove over sweaty hands, which is actually taxing as well as causes discomfort.

The document US2014/289931 A1 discloses a detachable bi-layered glove.

Meanwhile, implementation of double gloving in the healthcare field was influenced by various factors such as but not limited to surgical procedure, risk status of patient, abrasion on the hands of the surgical team and personal preference. Similarly, cross-contamination between human hands and food products was the main reason for the implementation of double gloving in the food industry.

However, one major setback faced by users in double gloving is that the users require additional time for donning the second glove over the first glove which will directly affect the efficiency of the users. Thus, from the above, it is obvious that the glove manufacturing industries are facing some challenges to overcome, for which an approach is developed to identify a detachable bi-layered glove and an appropriate method for users to conveniently use the same piece of glove for twice without the risk of contamination and the hassle of doffing and donning for twice.

### SUMMARY OF THE INVENTION

The present invention relates to a detachable glove comprising at least two layers that is separated by an adhesive layer, wherein the adhesive layer includes a wetting agent, a latex coagulating agent, a synthetic organic polymer and water. The present invention also relates to a chlorinated detachable bi-layered glove and method of manufacturing thereof. The present invention further relates to a polymer coated detachable bi-layered glove and method of manufacturing thereof. Still further, the present invention relates to a method of peeling a detachable bi-layered glove wherein the method includes (i) pulling cuff area of the outer glove layer until its circumference is detached from the inner glove layer and (ii) peeling off the outer glove layer once the full circumference of the outer glove layer is detached from the inner glove layer to yield the inner glove layer for further usage.

Additional aspects, features and advantages of the invention will become apparent to those skilled in the art upon consideration of the following detailed description of preferred embodiments of the invention.

### BRIEF DESCRIPTION OF THE ACCOMPANYING DRAWINGS

The present invention will be fully understood from the detailed description given herein below and the accompanying drawings which are given by way of illustration only, and thus are not limitative of the present invention, wherein:
In the appended drawings:
**Figure 1** is a flowchart showing the steps involved in preparing the chlorinated detachable bi-layered glove of the present invention.
**Figure 2** is a flowchart showing the steps involved in preparing the polymer coated detachable bi-layered glove of the present invention.
**Figure 3a** is an illustrative figure of a glove with shorter inner glove layer and longer outer glove layer of the detachable bi-layered glove of the present invention.
**Figure 3b** is an illustrative figure showing the method of detaching the longer outer glove layer from the shorter inner glove layer of the detachable bi-layered glove of the present invention.
**Figure 3c** is an illustrative figure showing the method of peeling off the longer outer glove layer from the shorter inner glove layer of the detachable bi-layered glove of the present invention.
**Figure 3d** is an illustrative figure showing the shorter inner glove layer of the bi-layered glove of the present invention.
**Figure 4a** is an illustrative figure of a glove with longer inner glove layer and shorter outer glove layer of the detachable bi-layered glove of the present invention.
**Figure 4b** is an illustrative figure showing the method of detaching the shorter outer glove layer from the longer inner glove layer of the detachable bi-layered glove of the present invention.
**Figure 4c** is an illustrative figure showing the method of peeling off the shorter outer glove layer from the longer inner glove layer of the detachable bi-layered glove of the present invention.
**Figure 4d** is an illustrative figure showing the longer inner glove layer of the bi-layered glove of the present invention.

### DETAILED DESCRIPTION OF THE INVENTION

Detailed description of preferred embodiments of the present invention is disclosed herein. It should be understood, however, that the embodiments are merely exemplary of the present invention, which is solely defined by the claims. The numerical data or ranges used in the specification are not to be construed as limiting.

The present invention relates to a detachable bi-layered glove and method of manufacturing thereof, in particular the present invention relates to a detachable bi-layered but not limited to any glove selected from the group consisting of acrylonitrile butadiene rubber (NBR) glove, natural rubber (NR) glove, polychloroprene (CR) rubber glove, polyisoprene (PI) rubber glove, styrene butadiene rubber (SBR) glove, butadiene copolymers rubber glove, polyurethane (PU) glove, polyvinyl chloride (PVC) glove, thermoplastic elastomer (TPE) glove, vinyl acetate ethylene (VAE) glove and mixtures thereof and method of manufacturing thereof which provide users the convenience of using same piece of glove for twice without the risk of contamination and the hassle of doffing and donning for twice.

For the purpose of this invention, the term "bi-layered" can also be construed as two-layered or double-layered which is made of an outer glove layer and an inner glove layer. Simply, bi-layered glove is comprising an outer glove layer and an inner glove layer. For the purpose of this invention, the term "detachable" can also be construed as removable and/or peel off. Further, the term "detachable" is associated to the action of peeling off the outer glove layer from the inner glove layer.

First embodiment of the present invention discusses on detachable bi-layered glove. The detachable bi-layered glove is comprising an outer glove layer and an inner glove layer that is separated by an adhesive layer. The glove may be any glove selected from the group consisting of acrylonitrile butadiene rubber (NBR) glove, natural rubber (NR) glove and polychloroprene (CR) rubber glove, polyisoprene (PI) rubber glove, styrene butadiene rubber (SBR) glove, butadiene copolymers rubber glove, polyurethane (PU) glove, polyvinyl chloride (PVC) glove, thermoplastic elastomer (TPE) glove, vinyl acetate ethylene (VAE) glove and mixtures thereof.

Alternatively, the present invention may also be applied on detachable multi-layered glove. The multi-layered glove is of at least 3 layers. The layers are separated by an adhesive layer.

The latex to manufacture the glove may be obtained from any commercially available latex suppliers. The latexes are used as 100 phr (also referred to as parts per hundred rubber), wherein the parts per hundred rubber is used as a basis for amounting other components.

The adhesive layer includes at least:
wetting agent, wherein the wetting agent is used in an amount ranging between 0.40% to 0.80%, preferably 0.60% to 0.70% by weight of the adhesive layer, wherein the wetting agent is selected from the group consisting of non-ionic surfactant, blends of non-ionic surfactant and mixtures thereof. The non-ionic surfactant is selected from the group consisting of fatty acid ethoxylate, lauric acid ethoxylate, oleic acid ethoxylate, other similar compounds and mixtures thereof. The blends of non-ionic surfactant are polysiloxane surfactant which is selected from the group consisting of methyl (propylhydroxide ethoxylated) bis (trimethylsiloxy) silane, other similar compounds and mixtures thereof. The wetting agent is preferably fatty acid ethoxylate. The wetting agent serves to improve wettability of the adhesive layer;
latex coagulating agent, wherein the latex coagulating agent is used in an amount ranging between 17.00% to 20.00%, preferably 17.50% to 19.50% by weight of the adhesive layer. The latex coagulating agent is selected from the group consisting of calcium nitrate, zinc nitrate, calcium chloride and mixtures thereof, preferably calcium nitrate. The latex coagulating agent serves to coagulate latex dispersion;
synthetic organic polymer, wherein the synthetic organic polymer is used in an amount ranging between 10.00% to 14.00%, preferably 12.00% to 13.00% by weight of the adhesive layer. The synthetic organic polymer is selected from the group consisting of styrene acrylic copolymer emulsion, polymethyl methacrylate emulsion, low-density polyethylene emulsion, high-density polyethylene emulsion, polypropylene emulsion, polystyrene emulsion, polyurethane emulsion and mixtures thereof, preferably styrene acrylic copolymer emulsion or polymethyl methacrylate emulsion, still preferably styrene acrylic copolymer emulsion. The synthetic organic polymer serves to increase block resistance for efficient peeling off the outer glove layer from the inner glove layer; and
water, wherein the water is used in an amount ranging between 65.20% to 72.60%, preferably 66.80% to 69.90% by weight of the adhesive layer. The water serves as diluent.

Table 1 shows chemical components and compositions thereof (as mentioned above) used in preparing the adhesive layer.

**Table 1: Chemical components and compositions thereof to prepare the adhesive layer**

| **Chemicals** | **Working Range (Weight %)** | **Preferred range (Weight %)** |
|---|---|---|
| Wetting agent | 0.40% to 0.80% | 0.60% to 0.70% |
| Latex coagulating agent | 17.00% to 20.00% | 17.50% to 19.50% |
| Synthetic organic polymer | 10.00% to 14.00% | 12.00% to 13.00% |
| Diluent | 65.20% to 72.60% | 66.80% to 69.90% |

The outer glove layer and the inner glove layer are in different lengths for easy removal. Simply, if the outer glove layer is longer, then the inner glove layer would be shorter and vice versa. The longer glove layer has a standard length ranging between 270 mm to 290 mm, whereas the shorter glove layer has a standard length ranging between 230 mm to 250 mm.

Second embodiment of the present invention discusses on the method to prepare the detachable bi-layered glove (as stated above). The detachable bi-layered glove may be a chlorinated and/or polymer coated detachable bi-layered glove.

### Chlorinated detachable bi-layered glove

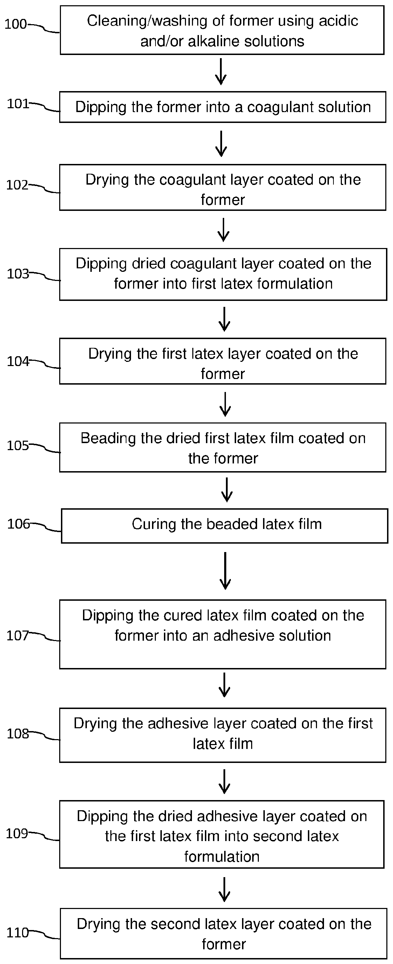

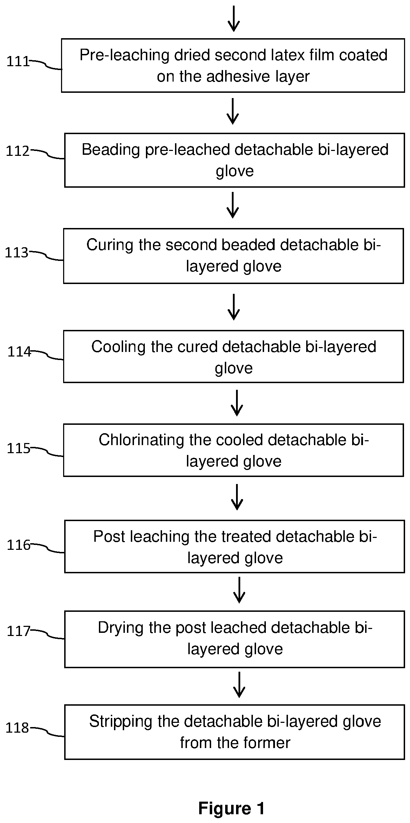

Referring to the accompanying drawing, **Figure 1** is a flowchart showing the method of preparing the chlorinated detachable bi-layered glove, wherein the method comprises the steps of:
i. cleaning/washing former firstly with acidic solution at a temperature ranging between 50°C to 70°C for a duration ranging between 3 seconds to 10 seconds, preferably between 5 seconds to 7 seconds, secondly (optionally) water rinsing at a temperature ranging between 50°C to 70°C for a duration ranging between 3 seconds to 10 seconds, preferably between 5 seconds to 7 seconds, followed by cleaning/washing with alkaline solution at a temperature ranging between 50°C to 70°C for a duration ranging between 5 seconds to 15 seconds, preferably between 10 seconds to 12 seconds and lastly water rinsing at a temperature ranging between 55°C to 75°C for a duration ranging between 5 seconds to 15 seconds, preferably between 10 seconds to 12 seconds to produce cleaned former **(100);**
ii. dipping the former obtained in step (i) into a coagulant solution to produce a coagulant layer coated on the former, wherein the dipping is carried out at a temperature ranging between 50°C to 70°C, preferably between 60°C to 65°C for a duration ranging between 8 seconds to 12 seconds, preferably between 9 seconds to 10 seconds, wherein the coagulant solution is any conventional coagulant solution **(101);**
iii. drying the coagulant layer coated on the former obtained in step (ii) to produce a dried coagulant layer coated on the former, wherein the drying is carried out at a temperature ranging between 100°C to 180°C, preferably between 130°C to 150°C for a duration ranging between 50 seconds to 180 seconds, preferably between 60 seconds to 120 seconds **(102);**
iv. dipping the dried coagulant layer coated on the former obtained in step (iii) into first latex formulation to produce a first latex layer coated on the former (which is the outer glove layer of the detachable bi-layered glove), wherein the dipping is carried out at a temperature ranging between 50°C to 70°C, preferably between 60°C to 65°C for a duration ranging between 8 seconds to 12 seconds, preferably between 9 seconds to 10 seconds **(103);**
v. drying the first latex layer coated on the former obtained in step (iv) to produce a dried first latex film coated on the former, wherein the drying is carried out at a temperature ranging between 100°C to 150°C, preferably between 120°C to 140°C for a duration ranging between 50 seconds to 180 seconds, preferably between 60 seconds to 120 seconds **(104);**
vi. beading the dried first latex film coated on the former obtained in step (v) to produce a beaded latex film coated on the former, wherein the beading is performed using any conventional beading carpet or beading brush found in the production line **(105);**
vii. curing the beaded latex film coated on the former obtained in step (vi) to produce a cured latex film coated on the former, wherein the curing is carried out at a temperature ranging between 100°C to 150°C, preferably between 120°C to 140°C for a duration ranging between 8 minutes to 20 minutes preferably between 10 minutes to 15 minutes **(106);**
viii. dipping the cured latex film coated on the former obtained in step (vii) into an adhesive solution to produce an adhesive layer coated on the first latex film, wherein the dipping is carried out at a temperature ranging between 50°C to 70°C, preferably between 60°C to 65°C for a duration ranging between 8 seconds to 12 seconds, preferably between 9 seconds to 10 seconds **(107);**
ix. drying the adhesive layer coated on the first latex film obtained in step (viii) to produce a dried adhesive layer coated on the first latex film, wherein the drying is carried out at a temperature ranging between 100°C to 180°C, preferably between 130°C to 150°C for a duration ranging between 50 seconds to 180 seconds, preferably between 60 seconds to 120 seconds **(108);**
x. dipping the dried adhesive layer coated on the first latex film obtained in step (ix) into second latex formulation to produce a second latex layer coated on the adhesive layer (which is the inner glove layer of the detachable bi-layered glove), wherein the dipping is carried out at a temperature ranging between 50°C to 70°C, preferably between 60°C to 65°C for a duration ranging between 8 seconds to 12 seconds, preferably between 9 seconds to 10 seconds **(109);**
xi. drying the second latex layer coated on the adhesive layer obtained in step (x) to produce a dried second latex film coated on the adhesive layer wherein the drying is carried out at a temperature ranging between 100°C to 150°C, preferably between 120°C to 140°C for a duration ranging between 50 seconds to 180 seconds, preferably between 60 seconds to 120 seconds (**110**);
xii. pre-leaching the dried second latex film coated on the adhesive layer obtained in step (xi) to produce a pre-leached detachable bi-layered latex film coated on the former, wherein the pre-leaching is carried out at a temperature ranging between 50°C to 70°C, preferably between 55°C to 60°C for a duration ranging between 15 seconds to 40 seconds, preferably between 20 seconds to 30 seconds **(111);**
xiii. beading the pre-leached detachable bi-layered latex film coated on the former obtained in step (xii) wherein the beading is performed using any conventional beading carpet or beading brush found in the production line to produce a beaded detachable bi-layered latex film on the surface **(112);**
xiv. curing the beaded detachable bi-layered latex film coated on the former obtained in step (xiv) to produce a cured detachable bi-layered glove coated on the former, wherein the curing is carried out at a temperature ranging between 110°C to 150°C, preferably between 120°C to 140°C for a duration ranging between 8 minutes to 20 minutes, preferably between 10 minutes to 15 minutes **(113);**
xv. cooling the cured detachable bi-layered glove coated on the former obtained in step (xiv) to produce a cooled detachable bi-layered glove coated on the former, wherein the cooling is carried out to bring down the temperature to a temperature ranging between 23°C to 30°C, preferably between 25°C to 27°C for a duration ranging between 8 seconds to 20 seconds, preferably between 10 seconds to 15 seconds **(114);**
xvi. chlorinating the cooled detachable bi-layered glove coated on the former obtained in step (xv) to produce a chlorine treated detachable bi-layered glove coated on the former, wherein the chlorination is carried out at a temperature ranging between 23°C to 30°C, preferably between 25°C to 27°C for a duration ranging between 8 seconds to 20 seconds, preferably between 10 seconds to 15 seconds **(115);**
xvii. post-leaching the chlorine treated detachable bi-layered glove coated on the former obtained in step (xvi) to produce a post-leached detachable bi-layered glove coated on the former, wherein the post-leaching is carried out at a temperature ranging between 50°C to 70°C, preferably between 55°C to 60°C for a duration ranging between 15 seconds to 40 seconds, preferably between 20 seconds to 30 seconds **(116);**
xviii. drying the post-leached detachable bi-layered glove coated on the former obtained in step (xvii) to produce the chlorinated detachable bi-layered glove, wherein the drying is carried out at a temperature ranging between 100°C to 180°C, preferably 130°C to 150°C for a duration ranging between 50 seconds to 180 seconds, preferably between 60 seconds to 120 seconds **(117);**
xix. stripping the chlorinated detachable bi-layered glove from the former **(118),** and
wherein the first and second latex formulations and resultant gloves thereof are of identical formulations, details of which is described below, wherein the first and the second gloves are in different lengths, wherein the longer glove has a standard length ranging between 270 mm to 290 mm, wherein the shorter glove has a standard length ranging between 230 mm to 250 mm.

### Polymer coated detachable bi-lavered glove

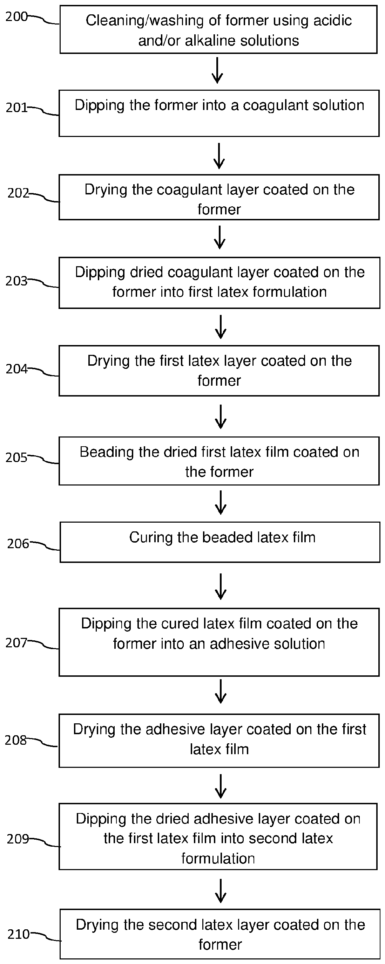

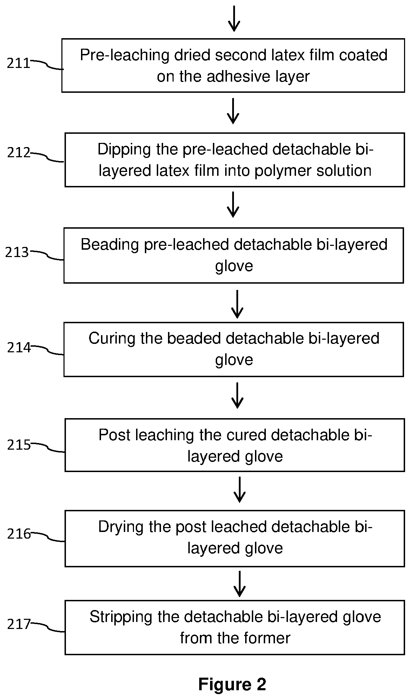

Referring to the accompanying drawing, **Figure 2** is a flowchart showing the method of preparing the polymer coated detachable bi-layered glove, wherein the method comprises the steps of:
i. cleaning/washing former firstly with acidic solution at a temperature ranging between 50°C to 70°C for a duration ranging between 3 seconds to 10 seconds, preferably between 5 seconds to 7 seconds, secondly (optionally) water rinsing at a temperature ranging between 50°C to 70°C for a duration ranging between 3 seconds to 10 seconds, preferably between 5 seconds to 7 seconds, followed by cleaning/washing with alkaline solution at a temperature ranging between 50°C to 70°C for a duration ranging between 5 seconds to 15 seconds, preferably between 10 seconds to 12 seconds and lastly water rinsing at a temperature ranging between 55°C to 75°C for a duration ranging between 5 seconds to 15 seconds, preferably between 10 seconds to 12 seconds to produce cleaned former **(200);**
ii. dipping the cleaned former obtained in step (i) into a coagulant solution to produce a coagulant layer coated on the former, wherein the dipping is carried out at a temperature ranging between 50°C to 70°C, preferably between 60°C to 65°C for a duration ranging between 8 seconds to 12 seconds, preferably between 9 seconds to 10 seconds, wherein the coagulant solution is any conventional coagulant solution **(201);**
iii. drying the coagulant layer coated on the former obtained in step (ii) to produce a dried coagulant layer coated on the former, wherein the drying is carried out at a temperature ranging between 100°C to 180°C, preferably between 130°C to 150°C for a duration ranging between 50 seconds to 180 seconds, preferably between 60 seconds to 120 seconds **(202);**
iv. dipping the dried coagulant layer coated on the former obtained in step (iii) into first latex formulation to produce a first latex layer coated on the former (which is the outer glove layer of the detachable bi-layered glove), wherein the dipping is carried out at a temperature ranging between 50°C to 70°C, preferably between 60°C to 65°C for a duration ranging between 8 seconds to 12 seconds, preferably between 9 seconds to 10 seconds **(203);**
v. drying the first latex layer coated on the former obtained in step (iv) to produce a dried first latex film coated on the former, wherein the drying is carried out at a temperature ranging between 100°C to 150°C, preferably between 120°C to 140°C for a duration ranging between 50 seconds to 180 seconds, preferably between 60 seconds to 120 seconds **(204);**
vi. beading the dried first latex film coated on the former obtained in step (v) to produce a beaded latex film coated on the former, wherein the beading is performed using any conventional beading carpet or beading brush found in the production line **(205);**
vii. curing the beaded latex film coated on the former obtained in step (vi) to produce a cured latex film coated on the former, wherein the curing is carried out at a temperature ranging between 100°C to 150°C, preferably between 120°C to 140°C for a duration ranging between 8 minutes to 20 minutes, preferably between 10 minutes to 15 minutes **(206);**
viii. dipping the cured latex film coated on the former obtained in step (vii) into an adhesive solution to produce an adhesive layer coated on the first latex film, wherein the dipping is carried out at a temperature ranging between 50°C to 70°C, preferably between 60°C to 65°C for a duration ranging between 8 seconds to 12 seconds, preferably between 9 seconds to 10 seconds **(207);**
ix. drying the adhesive layer coated on the first latex film obtained in step (viii) to produce a dried adhesive layer coated on the first latex film, wherein the drying is carried out at a temperature ranging between 100°C to 180°C, preferably between 130°C to 150°C for a duration ranging between 50 seconds to 180 seconds, preferably between 60 seconds to 120 seconds **(208);**
x. dipping the dried adhesive layer coated on the first latex film obtained in step (ix) into second latex formulation to produce a second latex layer coated on the adhesive layer (which is the inner glove layer of the detachable bi-layered glove), wherein the dipping is carried out at a temperature ranging between 50°C to 70°C, preferably between 60°C to 65°C for a duration ranging between 8 seconds to 12 seconds, preferably between 9 seconds to 10 seconds **(209);**
xi. drying the second latex layer coated on the adhesive layer obtained in step (x) to produce a dried second latex film coated on the adhesive layer wherein the drying is carried out at a temperature ranging between 100°C to 150°C, preferably between 120°C to 140°C for a duration ranging between 50 seconds to 180 seconds, preferably between 60 seconds to 120 seconds (**210**);
xii. pre-leaching the dried second latex film coated on the adhesive layer obtained in step (xi) to produce a pre-leached detachable bi-layered latex film coated on the former, wherein the pre-leaching is carried out at a temperature ranging between 50°C to 70°C, preferably between 55°C to 60°C for a duration ranging between 15 seconds to 40 seconds, preferably between 20 seconds to 30 seconds **(211);**
xiii. dipping the pre-leached detachable bi-layered latex film obtained in step (xii) into polymer solution to produce a polymer coated detachable bi-layered latex film, wherein the dipping is carried out at a temperature ranging between 25°C to 45°C, preferably between 35°C to 40°C for a duration ranging between 8 seconds to 15 seconds, preferably between 10 seconds to 12 seconds **(212);**
xiv. beading the polymer coated detachable bi-layered latex film coated on the former obtained in step (xiii), wherein the beading is performed using any conventional beading carpet or beading brush found in the production line to produce beaded a detachable bi-layered latex film on the surface **(213);**
xv. curing the beaded detachable bi-layered latex film coated on the former obtained in step (xiv) to produce a cured detachable bi-layered glove coated on the former, wherein the curing is carried out at a temperature ranging between 110°C to 150°C, preferably between 120°C to 140°C for a duration ranging between 8 minutes to 20 minutes, preferably between 10 minutes to 15 minutes **(214);**
xvi. post-leaching the cured detachable bi-layered glove coated on the former obtained in step (xv) to produce post-leached detachable bi-layered glove coated on the former, wherein the post-leaching is carried out at a temperature ranging between 50°C to 70°C, preferably between 55°C to 60°C for a duration ranging between 15 seconds to 40 seconds, preferably between 20 seconds to 30 seconds **(215);**
xvii. drying the post-leached detachable bi-layered glove coated on the former obtained in step (xvi) to produce the polymer coated detachable bi-layered glove, wherein the drying is carried out at a temperature ranging between 100°C to 180°C, preferably 130°C to 150°C for a duration ranging between 50 seconds to 180 seconds, preferably between 60 seconds to 120 seconds **(216);**
xviii. stripping the polymer coated detachable bi-layered glove from the former **(217),** and
wherein the first and second latex formulations and resultant gloves thereof are of identical formulations, details of which is described below, wherein the first and the second gloves are in different lengths, wherein the longer glove has a standard length ranging between 270 mm to 290 mm, wherein the shorter glove has a standard length ranging between 230 mm to 250 mm.

The coagulant layer includes:
wetting agent (such as but not limited to fatty acid ethoxylate);
former release agent (such as but not limited to potassium stearate, calcium stearate and calcium carbonate);
latex coagulating agent (such as but not limited to calcium nitrate);
pH adjuster (such as but not limited to ammonia); and
diluent ( such as but not limited to water).

Tables 3, 5 and 7 show chemical components (as listed above) and compositions thereof used in preparing the coagulant layer that is in accordance to the latex formulations to be prepared.

The first and the second latex formulations include:
latex (such as but not limited to acrylonitrile butadiene rubber (NBR), natural rubber (NR) and polychloroprene (CR));
dispersing agent (such as but not limited to disodium N-octadecylsulphosuccinamate, salt of naphthalene sulphonic acid, and sodium silicaflouride aqueous dispersion);
stabilizer (such as but not limited to sodium lauryl sulphate and monosodium salt of sulphated methyl oleate);
pH adjuster (such as but not limited to ammonia, sodium hydroxide, potassium hydroxide);
accelerator (such as but not limited to zinc dibutyldithiocarbamate and zinc diethyldithiocarbamate);
crosslinking agent (such as but not limited to sulphur (primary) and/or zinc oxide (secondary));
pigment (such as but not limited to titanium dioxide);
filler, reinforcing agent (such as but not limited to silicate-based filler or calcium carbonate);
antioxidant (such as but not limited to polymeric phenolic);
mould release agent (such as but not limited to blend of paraffin wax-emulsion;
antifoaming agent (such as but not limited to methylcyclotetrasiloxane and/or dimethyl siloxane); and
wetting agent (such as but not limited to t-octylphenoxypolyethoxyethanol and/or polyethylene glycol sorbitan monostearate).

Tables 2, 4 to 6 show chemical components (as listed above) and compositions thereof used in preparing the first and the second latex formulations.

**Table 2: Chemical components and compositions thereof to prepare the first and the second latex formulations of NBR**

| **Chemical** | **Working range (*phr)** |
|---|---|
| NBR | 100.00 |
| Dispersing agent | 0.60 to 1.40 |
| pH adjuster | 1.80 to 2.60 |
| Primary crosslinking agent | 2.00 to 4.00 |
| Secondary crosslinking agent | 0.30 to 1.00 |
| Accelerator | 0.60 to 1.50 |
| Pigment | 0.60 to 1.50 |
| Antifoaming agent and wetting agent | 0.02 to 0.07 |
| Filler/reinforcing agent | 2.00 to 8.00 |

| | |
|---|---|
| *parts per hundred rubber | |

**Table 3: Chemical components and compositions thereof to prepare the coagulant layer for NBR latex formulations**

| **Chemical** | **Working range (Weight %)** |
|---|---|
| Wetting agent | 0.050 to 0.250 |
| Former release agent | 1.000 to 3.000 |
| Latex coagulating agent | 8.000 to 12.000 |
| pH adjuster | 0.025 to 0.050 |
| Diluent | 84.700 to 90.925 |

**Table 4: Chemical components and compositions thereof to prepare the first and the second latex formulations of NR**

| **Chemical** | **Working range (*phr)** |
|---|---|
| NR | 100.00 |
| pH adjuster | 0.05 to 0.40 |
| Filler/reinforcing agent | 35.00 to 55.00 |
| Accelerator | 0.10 to 0.30 |
| Primary crosslinking agent | 1.00 to 2.00 |
| Secondary crosslinking agent | 0.70 to 1.50 |
| Antifoaming agent | 0.03 to 0.20 |
| Mould release agent | 0.60 to 1.50 |
| Dispersing agent | 0.10 to 0.30 |

| | |
|---|---|
| *parts per hundred rubber | |

**Table 5: Chemical components and compositions thereof to prepare the coagulant layer for NR latex formulations**

| **Chemical** | **Working range (Weight %)** |
|---|---|
| Former release agent | 3.800 to 5.000 |
| Latex coagulating agent | 3.000 to 8.000 |
| Wetting agent | 0.050 to 0.250 |
| pH adjuster | 0.025 to 0.050 |
| Diluent | 86.700 to 93.125 |

**Table 6: Chemical components and compositions thereof to prepare the first and the second latex formulations of CR**

| **Chemical** | **Working range (*phr)** |
|---|---|
| CR | 100.00 |
| Wetting agent | 0.05 to 0.15 |
| Stabilizer | 0.30 to 0.70 |
| Dispersing agent | 6.00 to 10.00 |
| Crosslinking agent | 0.70 to 1.50 |
| Antioxidant | 0.20 to 0.70 |
| Pigment | 0.30 to 0.80 |

| | |
|---|---|
| *parts per hundred rubber | |

**Table 7: Chemical components and compositions thereof to prepare the first coagulant layer for CR latex formulations**

| **Chemical** | **Working range (Weight %)** |
|---|---|
| Former release agent | 3.000 to 4.000 |
| Wetting agent | 0.004 to 0.008 |
| Latex coagulating agent | 10.000 to 18.000 |
| pH adjuster | 0.025 to 0.050 |
| Diluent | 77.942 to 86.971 |

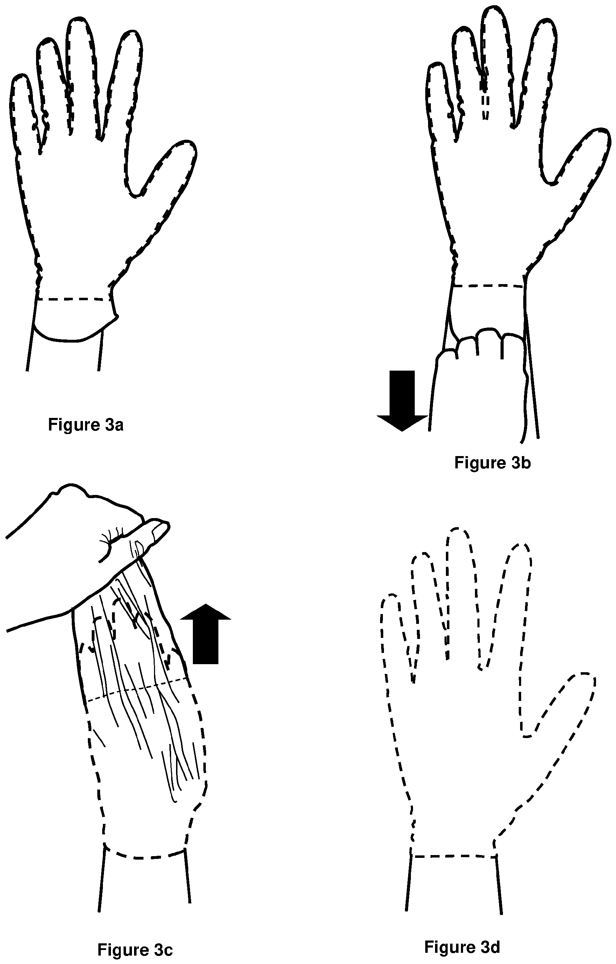

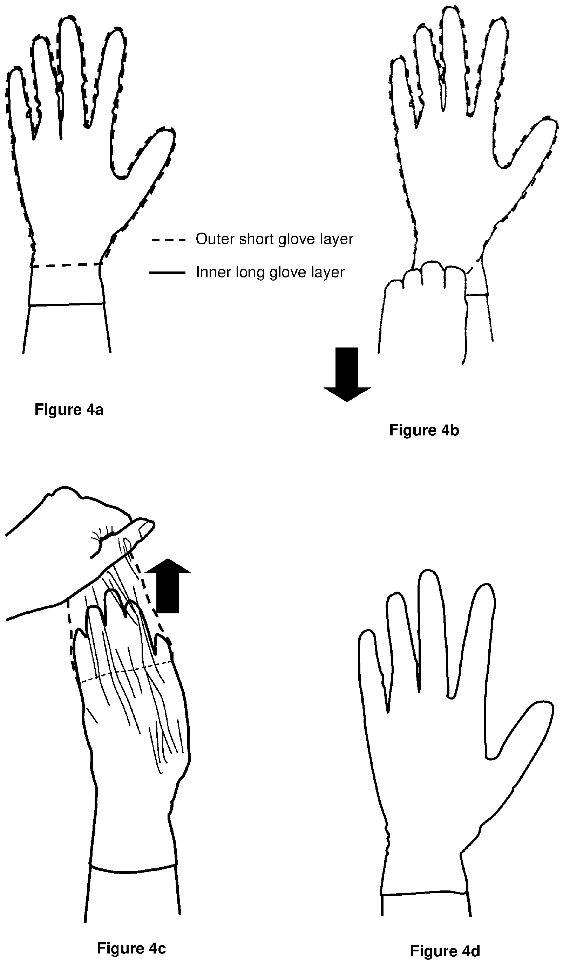

Third embodiment of the present invention discusses on method of peeling the outer glove layer of the detachable bi-layered glove from the inner glove layer of the detachable bi-layered glove. Referring to the accompanying drawings, **Figures 3a to 3d** as well as **Figures 4a to 4d** are illustrative figures showing the method of peeling off the outer glove layer of the detachable bi-layered glove from the inner glove layer of the detachable bi-layered glove, wherein the method comprises the steps of:
i. pulling cuff area of the outer glove layer until its full circumference is detached from the inner glove layer and
ii. peeling off the outer glove layer once the full circumference of the outer glove layer is detached from the inner glove layer to yield the inner glove layer for further usage.

The following example is constructed to illustrate the present invention in a nonlimiting sense.

A detachable bi-layered glove of the present invention is comprising an outer glove layer and an inner glove layer that is separated by an adhesive layer, wherein the gloves are prepared using latex formulations as described in Tables 2, 4 and 6 adopting a method commonly known in the glove manufacturing industry, wherein the adhesive layer is as described in Table 1 and wherein the outer and the inner gloves are in different lengths, wherein the longer glove has a standard length ranging between 270 mm to 290 mm and wherein the shorter glove has a standard length ranging between 230 mm to 250 mm.

### Test results for all the gloves

Table 8 shows the survey results from 20 respondents on the peeling efficiency of the detachable bi-layered glove of the present invention.

**Table 8: Peeling efficiency of the detachable bi-layered glove of the present invention**

| **Detachable glove latex type** | **Peeling efficiency** |
|---|---|
| Acrylonitrile butadiene rubber (NBR) | Very good |
| Polychloroprene rubber (CR) | Very good |
| Natural rubber (NR) | Good |

Based on user's experience, it is evident that the users are finding the peeling efficiencies are very good for both the NBR and the CR as compared to NR detachable bi-layered gloves.

Tables 9 and 10 shows the survey results obtained from 20 respondents on peeling efficiencies of detachable bi-layered glove of the present invention when the inner layer and outer layer are in different lengths with different adhesive layers are applied between the detachable bi-layered glove of the present invention. The peeling efficiencies are rated from 1 to 3, wherein 1 signifies easy peeling, 2 signifies normal peeling and 3 signifies hard peeling.

**Table 9: Peeling efficiencies of the detachable bi-layered glove of the present invention when different adhesive layers are applied between the detachable bi-layered glove of the present invention with a longer outer layer and a shorter inner layer**

| Detachable glove latex type | Peeling efficiency | | Tearing during peeling of outer layer | |
|---|---|---|---|---|
| | Adhesive layer of the present invention | Conventional adhesive layer | Adhesive layer of the present invention | Conventional adhesive layer |
| Acrylonitrile butadiene rubber (NBR) | 1 | 3 | No | Yes |
| Polychloroprene rubber (CR) | 1 | 3 | No | Yes |
| Natural rubber (NR) | 2 | 3 | No | Yes |

**Table 10: Peeling efficiencies of the detachable bi-layered glove of the present invention when different adhesive layers are applied between the detachable bi-layered glove of the present invention with a shorter outer layer and a longer inner layer**

| Detachable glove latex type | Peeling efficiency | | Tearing during peeling of outer layer | |
|---|---|---|---|---|
| | Adhesive layer of the present invention | Conventional adhesive layer | Adhesive layer of the present invention | Conventional adhesive layer |
| Acrylonitrile butadiene rubber (NBR) | 1 | 3 | No | Yes |
| Polychloroprene rubber (CR) | 1 | 3 | No | Yes |
| Natural rubber (NR) | 2 | 3 | No | Yes |

Based on user's experience, it is evident that the users are finding the peeling efficiencies are good for all the detachable bi-layered gloves with adhesive layer of the present invention applied between the detachable bi-layered gloves.

As a whole, detachable bi-layered glove of the present invention is able to overcome the conventional shortcomings since the glove of the present invention is able to cater users to conveniently use the same piece of glove twice without the risk of contamination and without the hassle of doffing and donning twice.

The terminology used herein is for the purpose of describing particular example embodiments only and is not intended to be limiting. As used herein, the singular forms "a", "an" and "the" may be intended to include the plural forms as well, unless the context clearly indicates otherwise. The terms "comprises", "comprising", "including", and "having" are inclusive and therefore specify the presence of stated features, integers, steps, operations, elements, and/or components, but do not preclude the presence or addition of one or more other features, integers, steps, operations, elements, components, and/or groups thereof.

## Claims

1. A detachable bi-layered glove is comprising an outer glove layer and an inner glove layer that is separated by an adhesive layer, the glove being **characterized in that** the adhesive layer includes a wetting agent, a latex coagulating agent, a synthetic organic polymer and water, wherein the wetting agent is used in an amount ranging between 0.40% to 0.80% by weight of the adhesive layer, wherein the latex coagulating agent is used in an amount ranging between 17.00% to 20.00% by weight of the adhesive layer, wherein the synthetic organic polymer is used in an amount ranging between 10.00% to 14.00% by weight of the adhesive layer and wherein water is used in an amount ranging between 65.20% to 72.60% by weight of the adhesive layer.

2. The detachable bi-layered glove as claimed in claim 1, wherein the wetting agent is used in an amount ranging between 0.60% to 0.70% by weight of the adhesive layer.

3. The detachable bi-layered glove as claimed in claim 1, wherein the wetting agent is selected from the group consisting of non-ionic surfactant, blends of non-ionic surfactant and mixtures thereof.

4. The detachable bi-layered glove as claimed in claim 3, wherein the non-ionic surfactant is selected from the group consisting of fatty acid ethoxylate, lauric acid ethoxylate, oleic acid ethoxylate, other similar compounds and mixtures thereof.

5. The detachable bi-layered glove as claimed in claim 3, wherein the blends of non-ionic surfactant are polysiloxane surfactant which is selected from the group consisting of methyl (propylhydroxide ethoxylated) bis (trimethylsiloxy) silane, other similar compounds and mixtures thereof.

6. The detachable bi-layered glove as claimed in claim 1, wherein the latex coagulating agent is used in an amount ranging between 17.50% to 19.50% by weight of the adhesive layer.

7. The detachable bi-layered glove as claimed in claim 1, wherein the latex coagulating agent is selected from the group consisting of calcium nitrate, zinc nitrate, calcium chloride and mixtures thereof.

8. The detachable bi-layered glove as claimed in claim 1, wherein the synthetic organic polymer is used in an amount ranging between 12.00% to 13.00% by weight of the adhesive layer.

9. The detachable bi-layered glove as claimed in claim 1, wherein the synthetic organic polymer is selected from the group consisting of styrene acrylic copolymer emulsion, polymethyl methacrylate emulsion, low-density polyethylene emulsion, high-density polyethylene emulsion, polypropylene emulsion, polystyrene emulsion, polyurethane emulsion and mixtures thereof.

10. The detachable bi-layered glove as claimed in claim 1, wherein water is used in an amount ranging between 66.80% to 69.90% by weight of the adhesive layer.

11. A detachable multi-layered glove, wherein the multi-layered glove is of at least 3 layers, wherein the layers are separated by an adhesive layer, the glove being **characterized in that** the adhesive layer includes a wetting agent, a latex coagulating agent, a synthetic organic polymer and water, wherein the wetting agent is used in an amount ranging between 0.40% to 0.80% by weight of the adhesive layer, wherein the latex coagulating agent is used in an amount ranging between 17.00% to 20.00% by weight of the adhesive layer, wherein the synthetic organic polymer is used in an amount ranging between 10.00% to 14.00% by weight of the adhesive layer and wherein water is used in an amount ranging between 65.20% to 72.60% by weight of the adhesive layer.

12. A detachable bi-layered glove as claimed in claims 1 to 10, wherein the detachable bi-layered glove is a chlorinated glove.

13. A method of manufacturing a chlorinated detachable bi-layered glove, wherein the method comprises the steps of:
i. dipping a former into a coagulant solution to produce a coagulant layer coated on the former;
ii. drying the coagulant layer coated on the former obtained in step (i) to produce a dried coagulant layer coated on the former;
iii. dipping the dried coagulant layer coated on the former obtained in step (ii) into first latex formulation to produce a first latex layer coated on the former;
iv. drying the first latex layer coated on the former obtained in step (iii) to produce a dried first latex film coated on the former;
v. beading the dried first latex film coated on the former obtained in step (iv) to produce a beaded latex film coated on the former;
vi. curing the beaded latex film coated on the former obtained in step (v) to produce a cured latex film coated on the former;
vii. dipping the cured latex film coated on the former obtained in step (vi) into an adhesive solution to produce an adhesive layer coated on the first latex film;
viii. drying the adhesive layer coated on the first latex film obtained in step (vii) to produce a dried adhesive layer coated on the first latex film;
ix. dipping the dried adhesive layer coated on the first latex film obtained in step (viii) into second latex formulation to produce a second latex layer coated on the adhesive layer;
x. drying the second latex layer coated on the adhesive layer obtained in step (ix) to produce a dried second latex film coated on the adhesive layer;
xi. pre-leaching the dried second latex film coated on the adhesive layer obtained in step (x) to produce a pre-leached detachable bi-layered latex film coated on the former;
xii. beading the pre-leached detachable bi-layered latex film coated on the former obtained in step (xi) to produce a beaded detachable bi-layered latex film coated on the former;
xiii. curing the beaded detachable bi-layered latex film coated on the former obtained in step (xiii) to produce a cured detachable bi-layered glove coated on the former;
xiv. cooling the cured detachable bi-layered glove coated on the former obtained in step (xiii) to produce a cooled detachable bi-layered glove coated on the former;
xv. chlorinating the cooled detachable bi-layered glove coated on the former obtained in step (xiv) to produce a chlorine treated detachable bi-layered glove coated on the former;
xvi. post-leaching the chlorine treated detachable bi-layered glove coated on the former obtained in step (xv) to produce a post-leached detachable bi-layered glove coated on the former; and
xvii. drying the post-leached detachable bi-layered glove coated on the former obtained in step (xvi) to produce the chlorinated detachable bi-layered glove,
**characterized in that** the adhesive layer produced in the step (vii) includes a wetting agent, a latex coagulating agent, a synthetic organic polymer and water, wherein the wetting agent is used in an amount ranging between 0.40% to 0.80% by weight of the adhesive layer, wherein the latex coagulating agent is used in an amount ranging between 17.00% to 20.00% by weight of the adhesive layer, wherein the synthetic organic polymer is used in an amount ranging between 10.00% to 14.00% by weight of the adhesive layer and wherein water is used in an amount ranging between 65.20% to 72.60% by weight of the adhesive layer.

14. A detachable bi-layered glove as claimed in claims 1 to 10, wherein the detachable bi-layered glove is a polymer coated glove.

15. A method of manufacturing a polymer coated detachable bi-layered glove, wherein the method comprises the steps of:
i. dipping the former into a coagulant solution to produce a coagulant layer coated on the former;
ii. drying the coagulant layer coated on the former obtained in step (i) to produce a dried coagulant layer coated on the former;
iii. dipping the dried coagulant layer coated on the former obtained in step (ii) into first latex formulation to produce a first latex layer coated on the former;
iv. drying the first latex layer coated on the former obtained in step (iii) to produce a dried first latex film coated on the former;
v. beading the dried first latex film coated on the former obtained in step (iv) to produce a beaded latex film coated on the former;
vi. curing the beaded latex film coated on the former obtained in step (v) to produce a cured latex film coated on the former;
vii. dipping the cured latex film coated on the former obtained in step (vi) into an adhesive solution to produce an adhesive layer coated on the first latex film;
viii. drying the adhesive layer coated on the first latex film obtained in step (vii) to produce a dried adhesive layer coated on the first latex film;
ix. dipping the dried adhesive layer coated on the first latex film obtained in step (viii) into second latex formulation to produce a second latex layer coated on the adhesive layer;
x. drying the second latex layer coated on the adhesive layer obtained in step (ix) to produce a dried second latex film coated on the adhesive layer;
xi. pre-leaching the dried second latex film coated on the adhesive layer obtained in step (x) to produce a pre-leached detachable bi-layered latex film coated on the former;
xii. dipping the pre-leached detachable bi-layered latex film obtained in step (xi) into polymer solution to produce a polymer coated detachable bi-layered latex film;
xiii. beading the polymer coated detachable bi-layered latex film coated on the former obtained in step (xii) to produce a beaded detachable bi-layered latex film coated on the former;
xiv. curing the beaded detachable bi-layered latex film coated on the former obtained in step (xiii) to produce a cured detachable bi-layered glove coated on the former;
xv. post-leaching the cured detachable bi-layered glove coated on the former obtained in step (xiv) to produce a post-leached detachable bi-layered glove coated on the former;
xvi. drying the post-leached detachable bi-layered glove coated on the former obtained in step (xvi) to produce the polymer coated detachable bi-layered glove,
**characterized in that** the adhesive layer produced in the step (vii) includes a wetting agent, a latex coagulating agent, a synthetic organic polymer and water, wherein the wetting agent is used in an amount ranging between 0.40% to 0.80% by weight of the adhesive layer, wherein the latex coagulating agent is used in an amount ranging between 17.00% to 20.00% by weight of the adhesive layer, wherein the synthetic organic polymer is used in an amount ranging between 10.00% to 14.00% by weight of the adhesive layer and wherein water is used in an amount ranging between 65.20% to 72.60% by weight of the adhesive layer.

16. A method of peeling a detachable bi-layered glove as claimed in claims 1 to 15, wherein the method comprises the steps of:
i. pulling cuff area of the outer glove layer until its full circumference is detached from the inner glove layer; and
ii. peeling off the outer glove layer once the full circumference of the outer glove layer is detached from the inner glove layer to yield the inner glove layer for further usage.

## Patentansprüche

1. Trennbarer zweilagiger Handschuh, aufweisend eine äußere Handschuhschicht und eine innere Handschuhschicht, welche durch eine Adhäsionsschicht getrennt sind, wobei der Handschuh **dadurch gekennzeichnet ist, dass** die Adhäsionsschicht ein Benetzungsmittel, ein Latex-Koagulationsmittel, ein synthetisches organisches Polymer und Wasser enthält, wobei das Benetzungsmittel in einer Menge in einem Bereich zwischen 0,40 bis 0,80 Gewichts-% der Adhäsionsschicht verwendet wird, wobei das Latex-Koagulationsmittel in einer Menge in einem Bereich zwischen 17,00 bis 20,00 Gewichts-% der Adhäsionsschicht verwendet wird, wobei das synthetische organische Polymer in einer Menge in einem Bereich zwischen 10,00 bis 14,00 Gewichts-% der Adhäsionsschicht verwendet wird und wobei Wasser in einer Menge in einem Bereich zwischen 65,20 bis 72,60 Gewichts-% der Adhäsionsschicht verwendet wird.

2. Trennbarer zweilagiger Handschuh wie in Anspruch 1 beansprucht, wobei das Benetzungsmittel in einer Menge in einem Bereich zwischen 0,60 bis 0,70 Gewichts-% der Adhäsionsschicht verwendet wird.

3. Trennbarer zweilagiger Handschuh wie in Anspruch 1 beansprucht, wobei das Benetzungsmittel aus der Gruppe bestehend aus nicht-ionischen Tensiden, Gemischen von nicht-ionischen Tensiden und Mischungen davon ausgewählt ist.

4. Trennbarer zweilagiger Handschuh wie in Anspruch 3 beansprucht, wobei das nicht-ionische Tensid aus der Gruppe bestehend aus Fettsäureethoxylat, Laurinsäureethoxylat, Ölsäureethoxylat, anderen ähnlichen Verbindungen und Mischungen davon ausgewählt ist.

5. Trennbarer zweilagiger Handschuh wie in Anspruch 3 beansprucht, wobei die Gemische aus nicht-ionischen Tensiden Polysiloxan-Tenside sind, die aus der Gruppe bestehend aus Methyl(propylhydroxid-ethoxyliertes)bis(trimethylsiloxy)silan, anderen ähnlichen Verbindungen und Mischungen davon ausgewählt sind.

6. Trennbarer zweilagiger Handschuh wie in Anspruch 1 beansprucht, wobei das Latex-Koagulationsmittel in einer Menge in einem Bereich zwischen 17,50 bis 19,50 Gewichts-% der Adhäsionsschicht verwendet wird.

7. Trennbarer zweilagiger Handschuh wie in Anspruch 1 beansprucht, wobei das Latex-Koagulationsmittel aus der Gruppe bestehend aus Kalziumnitrat, Zinknitrat, Kalziumchlorid und Mischungen davon ausgewählt ist.

8. Trennbarer zweilagiger Handschuh wie in Anspruch 1 beansprucht, wobei das synthetische organische Polymer in einer Menge in einem Bereich zwischen 12,00 bis 13,00 Gewichts-% der Adhäsionsschicht verwendet wird.

9. Trennbarer zweilagiger Handschuh wie in Anspruch 1 beansprucht, wobei das synthetische organische Polymer aus der Gruppe bestehend aus Styrol-Acryl-Copolymer-Emulsion, Polymethylmethacrylat-Emulsion, Polyethylen-Emulsion niedriger Dichte, Polyethylen-Emulsion hoher Dichte, Polypropylen-Emulsion, Polystyrol-Emulsion, Polyurethan-Emulsion und Mischungen davon ausgewählt ist.

10. Trennbarer zweilagiger Handschuh wie in Anspruch 1 beansprucht, wobei Wasser in einer Menge in einem Bereich zwischen 66,80 bis 69,90 Gewichts-% der Adhäsionsschicht verwendet wird.

11. Trennbarer mehrlagiger Handschuh, wobei der mehrlagige Handschuh aus mindestens drei Lagen besteht, wobei die Lagen durch eine Adhäsionsschicht getrennt sind, wobei der Handschuh **dadurch gekennzeichnet ist, dass** die Adhäsionsschicht ein Benetzungsmittel, ein Latex-Koagulationsmittel, ein synthetisches organisches Polymer und Wasser enthält, wobei das Benetzungsmittel in einer Menge in einem Bereich zwischen 0,40 bis 0,80 Gewichts-% der Adhäsionsschicht verwendet wird, wobei das Latex-Koagulationsmittel in einer Menge in einem Bereich zwischen 17,00 bis 20,00 Gewichts-% der Adhäsionsschicht verwendet wird, wobei das synthetische organische Polymer in einer Menge in einem Bereich zwischen 10,00 bis 14,00 Gewichts-% der Adhäsionsschicht verwendet wird und wobei Wasser in einer Menge in einem Bereich zwischen 65,20 bis 72,60 Gewichts-% der Adhäsionsschicht verwendet wird.

12. Trennbarer zweilagiger Handschuh wie in den Ansprüchen 1 bis 10 beansprucht, wobei der abnehmbare zweilagige Handschuh ein chlorierter Handschuh ist.

13. Verfahren zur Herstellung eines chlorierten trennbaren zweilagigen Handschuhs, wobei das Verfahren die folgenden Schritte umfasst:
i. Eintauchen eines Formlings in eine Koagulationsmittellösung, um eine auf den Formling aufgebrachte Koagulationsschicht zu erzeugen;
ii. Trocknen der in Schritt (i) erhaltenen auf den Formling aufgebrachten Koagulationsschicht, um eine getrocknete auf den Formling aufgebrachte Koagulationsschicht zu erzeugen;
iii. Eintauchen der in Schritt (i) erhaltenen auf den Formling aufgebrachten getrockneten Koagulationsschicht in eine erste Latexrezeptur, um eine erste auf den Formling aufgebrachte Latexschicht zu erzeugen;
iv. Trocknen der in Schritt (iii) erhaltenen auf den Formling aufgebrachten ersten Latexschicht, um einen auf den Formling aufgebrachten getrockneten ersten Latexfilm zu erhalten;
v. Aufrauen des in Schritt (iv) erhaltenen auf den Formling aufgebrachten getrockneten ersten Latexfilms, um einen auf den Formling aufgebrachten aufgerauten Latexfilm zu erhalten;
vi. Aushärten des in Schritt (v) erhaltenen auf den Formling aufgebrachten aufgerauten Latexfilms, um einen auf den Formling aufgebrachten ausgehärteten Latexfilm zu erhalten;
vii. Eintauchen des in Schritt (vi) erhaltenen ausgehärteten auf den Formling aufgebrachten Latexfilms in eine Klebstofflösung, um eine auf den ersten Latexfilm aufgebrachte Adhäsionsschicht zu erzeugen;
viii. Trocknen der in Schritt (vii) erhaltenen auf den ersten Latexfilm aufgebrachten Adhäsionsschicht, um eine auf den ersten Latexfilm aufgebrachte getrocknete Adhäsionsschicht zu erhalten;
ix. Eintauchen der in Schritt (viii) erhaltenen auf den ersten Latexfilm aufgebrachten getrockneten Adhäsionsschicht in eine zweite Latexrezeptur, um eine auf die Adhäsionsschicht aufgebrachte zweite Latexschicht zu erzeugen;
x. Trocknen der in Schritt (ix) erhaltenen auf die Adhäsionsschicht aufgebrachten zweiten Latexschicht, um einen auf die Adhäsionsschicht aufgebrachten getrockneten zweiten Latexfilm zu erhalten;
xi. Vorlaugen des in Schritt (x) erhaltenen auf die Adhäsionsschicht aufgebrachten getrockneten zweiten Latexfilms, um einen auf den Formling aufgebrachten vorgelaugten trennbaren zweilagigen Latexfilm zu erhalten;
xii. Aufrauen des in Schritt (xi) erhaltenen auf den Formling aufgebrachten vorgelaugten trennbaren zweilagigen Latexfilms, um einen auf den Formling aufgebrachten aufgerauten trennbaren zweilagigen Latexfilm zu erhalten;
xiii. Aushärten des in Schritt (xiii) [*gemeint: xii*] erhaltenen auf den Formling aufgebrachten aufgerauten trennbaren zweilagigen Latexfilms, um einen auf den Formling aufgebrachten ausgehärteten trennbaren zweilagigen Handschuh zu erhalten;
xiv. Abkühlen des in Schritt (xiii) erhaltenen auf den Formling aufgebrachten ausgehärteten trennbaren zweilagigen Handschuhs, um einen auf den Formling aufgebrachten abgekühlten trennbaren zweilagigen Handschuh zu erhalten;
xv. Chlorieren des in Schritt (xiv) erhaltenen auf den Formling aufgebrachten abgekühlten trennbaren zweilagigen Handschuhs, um einen auf den Formling aufgebrachten chlorbehandelten trennbaren zweilagigen Handschuh zu erhalten;
xvi. Nachlaugen des in Schritt (xv) erhaltenen auf den Formling aufgebrachten chlorbehandelten trennbaren zweilagigen Handschuhs, um einen auf den Formling aufgebrachten nachgelaugten trennbaren zweilagigen Handschuh zu erhalten; und
xvii. Trocknen des in Schritt (xvi) erhaltenen auf den Formling aufgebrachten nachgelaugten trennbaren zweilagigen Handschuhs, um den chlorierten trennbaren zweilagigen Handschuh zu erhalten,
**dadurch gekennzeichnet, dass** die in Schritt (vii) erhaltene Adhäsionsschicht ein Benetzungsmittel, ein Latex-Koagulationsmittel, ein synthetisches organisches Polymer und Wasser enthält, wobei das Benetzungsmittel in einer Menge in einem Bereich zwischen 0,40 bis 0,80 Gewichts-% der Adhäsionsschicht verwendet wird, wobei das Latex-Koagulationsmittel in einer Menge in einem Bereich zwischen 17,00 bis 20,00 Gewichts-% der Adhäsionsschicht verwendet wird, wobei das synthetische organische Polymer in einer Menge in einem Bereich zwischen 10,00 bis 14,00 Gewichts-% der Adhäsionsschicht verwendet wird und wobei Wasser in einer Menge in einem Bereich zwischen 65,20 bis 72,60 Gewichts-% der Adhäsionsschicht verwendet wird.

14. Trennbarer zweilagiger Handschuh wie in den Ansprüchen 1 bis 10 beansprucht, wobei der trennbare zweilagige Handschuh ein polymerbeschichteter Handschuh ist.

15. Verfahren zur Herstellung eines polymerbeschichteten trennbaren zweilagigen Handschuhs, wobei das Verfahren die folgenden Schritte aufweist:
i. Eintauchen eines Formlings in eine Koagulationsmittellösung, um eine auf den Formling aufgebrachte Koagulationsschicht zu erzeugen;
ii. Trocknen der in Schritt (i) erhaltenen auf den Formling aufgebrachten Koagulationsschicht, um eine auf den Formling aufgebrachte getrocknete Koagulationsschicht zu erzeugen;
iii. Eintauchen der in Schritt (i) erhaltenen auf den Formling aufgebrachten getrockneten Koagulationsschicht in eine erste Latexrezeptur, um eine auf den Formling aufgebrachte erste Latexschicht zu erzeugen;
iv. Trocknen der in Schritt (iii) erhaltenen auf den Formling aufgebrachten ersten Latexschicht, um einen auf den Formling aufgebrachten getrockneten ersten Latexfilm zu erhalten;
v. Aufrauen des in Schritt (iv) erhaltenen auf den Formling aufgebrachten getrockneten ersten Latexfilms, um einen auf den Formling aufgebrachten aufgerauten Latexfilm zu erhalten;
vi. Aushärten des in Schritt (v) erhaltenen auf den Formling aufgebrachten aufgerauten Latexfilms, um einen auf den Formling aufgebrachten ausgehärteten Latexfilm zu erhalten;
vii. Eintauchen des in Schritt (vi) erhaltenen auf den Formling aufgebrachten ausgehärteten Latexfilms in eine Klebstofflösung, um eine auf den ersten Latexfilm aufgebrachte Adhäsionsschicht zu erzeugen;
viii. Trocknen der in Schritt (vii) erhaltenen auf den ersten Latexfilm aufgebrachten Adhäsionsschicht, um eine auf den ersten Latexfilm aufgebrachte getrocknete Adhäsionsschicht zu erhalten;
ix. Eintauchen der in Schritt (viii) erhaltenen auf den ersten Latexfilm aufgebrachten getrockneten Adhäsionsschicht in eine zweite Latexrezeptur, um eine auf die Adhäsionsschicht aufgebrachte zweite Latexschicht zu erzeugen;
x. Trocknen der in Schritt (ix) erhaltenen auf die Adhäsionsschicht aufgebrachten zweiten Latexschicht, um einen auf die Adhäsionsschicht aufgebrachten getrockneten zweiten Latexfilm zu erhalten;
xi. Vorlaugen des in Schritt (x) erhaltenen auf die Adhäsionsschicht aufgebrachten getrockneten zweiten Latexfilm, um einen auf den Formling aufgebrachten vorgelaugten trennbaren zweilagigen Latexfilm zu erhalten;
xii. Eintauchen des in Schritt (xi) erhaltenen vorgelaugten trennbaren zweilagigen Latexfilms in eine Polymerlösung, um einen polymerbeschichteten trennbaren zweilagigen Latexfilm zu erhalten;
xiii. Aufrauen des in Schritt (xii) erhaltenen auf den Formling aufgebrachten polymerbeschichteten trennbaren zweilagigen Latexfilms, um einen auf den Formling aufgebrachten aufgerauten trennbaren zweilagigen Latexfilm zu erhalten;
xiv. Aushärten des in Schritt (xiii) erhaltenen auf den Formling aufgebrachten aufgerauten trennbaren zweilagigen Latexfilms, um einen auf den Formling aufgebrachten ausgehärteten trennbaren zweilagigen Handschuh zu erhalten;
xv. Nachlaugen des in Schritt (xiv) erhaltenen auf den Formling aufgebrachten ausgehärteten trennbaren zweilagigen Handschuhs, um einen auf den Formling aufgebrachten nachgelaugten trennbaren zweilagigen Handschuh zu erhalten;
xvi. Trocknen des in Schritt (xvi) erhaltenen auf den Formling aufgebrachten nachgelaugten trennbaren zweilagigen Handschuhs, um den polymerbeschichteten trennbaren zweilagigen Handschuh zu erhalten,
**dadurch gekennzeichnet, dass** die in Schritt (vii) hergestellte Adhäsionsschicht ein Benetzungsmittel, ein Latex-Koagulationsmittel, ein synthetisches organisches Polymer und Wasser enthält, wobei das Benetzungsmittel in einer Menge in einem Bereich zwischen 0,40 bis 0,80 Gewichts-% der Adhäsionsschicht verwendet wird, wobei das Latex-Koagulationsmittel in einer Menge in einem Bereich zwischen 17,00 bis 20,00 Gewichts-% der Adhäsionsschicht verwendet wird, wobei das synthetische organische Polymer in einer Menge in einem Bereich zwischen 10,00 bis 14,00 Gewichts-% der Adhäsionsschicht verwendet wird und wobei Wasser in einer Menge in einem Bereich zwischen 65,20 bis 72,60 Gewichts-% der Adhäsionsschicht verwendet wird.

16. Verfahren zum Abziehen eines trennbaren zweilagigen Handschuhs wie in den Ansprüchen 1 bis 15 beansprucht, wobei das Verfahren die folgenden Schritte aufweist:
i. Ziehen am Bundbereich der äußeren Handschuhschicht, bis der gesamte Umfang von der inneren Handschuhschicht abgelöst ist; und
ii. Abziehen der äußeren Handschuhschicht, sobald der gesamte Umfang der äußeren Handschuhschicht von der inneren Handschuhschicht abgelöst ist, um die weitere Verwendbarkeit des Handschuhs zu erreichen.

## Revendications

1. Gant bicouche amovible comprenant une couche de gant extérieure et une couche de gant intérieure qui sont séparées par une couche adhésive, le gant étant **caractérisé en ce que** la couche adhésive comporte un agent mouillant, un agent de coagulation de latex, un polymère organique synthétique et de l'eau, dans lequel l'agent mouillant est utilisé en une quantité comprise entre 0,40 % et 0,80 % en poids de la couche adhésive, dans lequel l'agent de coagulation de latex est utilisé en une quantité comprise entre 17,00 % et 20,00 % en poids de la couche adhésive, dans lequel le polymère organique synthétique est utilisé en une quantité comprise entre 10,00 % et 14,00 % en poids de la couche adhésive et dans lequel de l'eau est utilisée en une quantité comprise entre 65,20 % et 72,60 % en poids de la couche adhésive.

2. Gant bicouche amovible selon la revendication 1, dans lequel l'agent mouillant est utilisé en une quantité comprise entre 0,60 % et 0,70 % en poids de la couche adhésive.

3. Gant bicouche amovible selon la revendication 1, dans lequel l'agent mouillant est choisi dans le groupe constitué d'un tensio-actif non ionique, de combinaisons de tensio-actif non ionique et leurs mélanges.

4. Gant bicouche amovible selon la revendication 3, dans lequel le tensio-actif non ionique est choisi dans le groupe constitué d'éthoxylate d'acide gras, d'éthoxylate d'acide laurique, d'éthoxylate d'acide oléique, d'autres composés similaires et un de leurs mélanges.

5. Gant bicouche amovible selon la revendication 3, dans lequel les combinaisons de tensio-actif non ionique sont un tensio-actif de polysiloxane qui est choisi dans le groupe constitué de méthyl (propylhydroxyde éthoxylé) bis (triméthylsiloxy) silane, d'autres composés similaires et un de leurs mélanges.

6. Gant bicouche amovible selon la revendication 1, dans lequel l'agent de coagulation de latex est utilisé en une quantité comprise entre 17,50 % et 19,50 % en poids de la couche adhésive.

7. Gant bicouche amovible selon la revendication 1, dans lequel l'agent de coagulation de latex est choisi dans le groupe constitué de nitrate de calcium, de nitrate de zinc, de chlorure de calcium et un de leurs mélanges.

8. Gant bicouche amovible selon la revendication 1, dans lequel le polymère organique synthétique est utilisé en une quantité comprise entre 12,00 % et 13,00 % en poids de la couche adhésive.

9. Gant bicouche amovible selon la revendication 1, dans lequel le polymère organique synthétique est choisi dans le groupe constitué d'une émulsion de copolymère de styrène-acrylique, d'une émulsion de polyméthacrylate de méthyle, d'une émulsion de polyéthylène basse densité, d'une émulsion de polyéthylène haute densité, d'une émulsion de polypropylène, d'une émulsion de polystyrène, d'une émulsion de polyuréthanne et un de leurs mélanges.

10. Gant bicouche amovible selon la revendication 1, dans lequel de l'eau est utilisée en une quantité comprise entre 66,80 % et 69,90 % en poids de la couche adhésive.

11. Gant multicouche amovible, dans lequel le gant multicouche est d'au moins 3 couches, dans lequel les couches sont séparées par une couche adhésive, le gant étant **caractérisé en ce que** la couche adhésive comporte un agent mouillant, un agent de coagulation de latex, un polymère organique synthétique et de l'eau, dans lequel l'agent mouillant est utilisé en une quantité comprise entre 0,40 % et 0,80 % en poids de la couche adhésive, dans lequel l'agent de coagulation de latex est utilisé en une quantité comprise entre 17,00 % et 20,00 % en poids de la couche adhésive, dans lequel le polymère organique synthétique est utilisé en une quantité comprise entre 10,00 % et 14,00 % en poids de la couche adhésive et dans lequel de l'eau est utilisée en une quantité comprise entre 65,20 % et 72,60 % en poids de la couche adhésive.

12. Gant bicouche amovible selon les revendications 1 à 10, dans lequel le gant bicouche amovible est un gant chloré.

13. Procédé de fabrication d'un gant bicouche amovible chloré, dans lequel le procédé comprend les étapes suivantes:
i. tremper une forme dans une solution de coagulant pour produire une couche de coagulant dont la forme est revêtue ;
ii. faire sécher la couche de coagulant dont la forme est revêtue obtenue à l'étape (i) pour produire une couche de coagulant séchée dont la forme est revêtue ;
iii. tremper la couche de coagulant séchée dont la forme est revêtue obtenue à l'étape (ii) dans une première formulation de latex pour produire une première couche de latex dont la forme est revêtue ;
iv. faire sécher la première couche de latex dont la forme est revêtue obtenue à l'étape (iii) pour produire un premier film de latex séché dont la forme est revêtue ;
v. faire perler le premier film de latex séché dont la forme est revêtue obtenu à l'étape (iv) pour produire un film de latex perlé dont la forme est revêtue ;
vi. faire durcir le film de latex perlé dont la forme est revêtue obtenu à l'étape (v) pour produire un film de latex durci dont la forme est revêtue ;
vii. tremper le film de latex durci dont la forme est revêtue obtenu à l'étape (vi) dans une solution adhésive pour produire une couche adhésive dont le premier film de latex est revêtu ;
viii. faire sécher la couche adhésive dont le premier film de latex est revêtu obtenue à l'étape (vii) pour produire une couche adhésive séchée dont le premier film de latex est revêtu ;
ix. tremper la couche adhésive séchée dont le premier film de latex est revêtu obtenue à l'étape (viii) dans une deuxième formulation de latex pour produire une deuxième couche de latex dont la couche adhésive est revêtue ;
x. faire sécher la deuxième couche de latex dont la couche adhésive est revêtue obtenue à l'étape (ix) pour produire un deuxième film de latex séché dont la couche adhésive est revêtue ;
xi. prélixivier le deuxième film de latex séché dont la couche adhésive est revêtue obtenu à l'étape (x) pour produire un film de latex bicouche amovible prélixivié dont la forme est revêtue ;
xii. faire perler le film de latex bicouche amovible prélixivié dont la forme est revêtue obtenu à l'étape (xi) pour produire un film de latex bicouche amovible perlé dont la forme est revêtue ;
xiii. faire durcir le film de latex bicouche amovible perlé dont la forme est revêtue obtenu à l'étape (xii) pour produire un gant bicouche amovible durci dont la forme est revêtue ;
xiv. refroidir le gant bicouche amovible durci dont la forme est revêtue obtenu à l'étape (xiii) pour produire un gant bicouche amovible refroidi dont la forme est revêtue ;
xv. chlorer le gant bicouche amovible refroidi dont la forme est revêtue obtenu à l'étape (xiv) pour produire un gant bicouche amovible traité au chlore dont la forme est revêtue ;
xvi. post-lixivier le gant bicouche amovible traité au chlore dont la forme est revêtue obtenu à l'étape (xv) pour produire un gant bicouche amovible post-lixivié dont la forme est revêtue ; et
xvii. faire sécher le gant bicouche amovible post-lixivié dont la forme est revêtue obtenu à l'étape (xvi) pour produire le gant bicouche amovible chloré,
**caractérisé en ce que** la couche adhésive produite à l'étape (vii) comporte un agent mouillant, un agent de coagulation de latex, un polymère organique synthétique et de l'eau, dans lequel l'agent mouillant est utilisé en une quantité comprise entre 0,40 % et 0,80 % en poids de la couche adhésive, dans lequel l'agent de coagulation de latex est utilisé en une quantité comprise entre 17,00 % et 20,00 % en poids de la couche adhésive, dans lequel le polymère organique synthétique est utilisé en une quantité comprise entre 10,00 % et 14,00 % en poids de la couche adhésive et dans lequel de l'eau est utilisée en une quantité comprise entre 65,20 % et 72,60 % en poids de la couche adhésive.

14. Gant bicouche amovible selon les revendications 1 à 10, dans lequel le gant bicouche amovible est un gant revêtu de polymère.

15. Procédé de fabrication d'un gant bicouche amovible revêtu de polymère, dans lequel le procédé comprend les étapes suivantes:
i. tremper la forme dans une solution de coagulant pour produire une couche de coagulant dont la forme est revêtue ;
ii. faire sécher la couche de coagulant dont la forme est revêtue obtenue à l'étape (i) pour produire une couche de coagulant séchée dont la forme est revêtue ;
iii. tremper la couche de coagulant séchée dont la forme est revêtue obtenue à l'étape (ii) dans une première formulation de latex pour produire une première couche de latex dont la forme est revêtue ;
iv. faire sécher la première couche de latex dont la forme est revêtue obtenue à l'étape (iii) pour produire un premier film de latex séché dont la forme est revêtue ;
v. faire perler le premier film de latex séché dont la forme est revêtue obtenu à l'étape (iv) pour produire un film de latex perlé dont la forme est revêtue ;
vi. faire durcir le film de latex perlé dont la forme est revêtue obtenu à l'étape (v) pour produire un film de latex durci dont la forme est revêtue ;
vii. tremper le film de latex durci dont la forme est revêtue obtenu à l'étape (vi) dans une solution adhésive pour produire une couche adhésive dont le premier film de latex est revêtu ;
viii. faire sécher la couche adhésive dont le premier film de latex est revêtu obtenue à l'étape (vii) pour produire une couche adhésive séchée dont le premier film de latex est revêtu ;
ix. tremper la couche adhésive séchée dont le premier film de latex est revêtu obtenue à l'étape (viii) dans une deuxième formulation de latex pour produire une deuxième couche de latex dont la couche adhésive est revêtue ;
x. faire sécher la deuxième couche de latex dont la couche adhésive est revêtue obtenue à l'étape (ix) pour produire un deuxième film de latex séché dont la couche adhésive est revêtue ;
xi. prélixivier le deuxième film de latex séché dont la couche adhésive est revêtue obtenu à l'étape (x) pour produire un film de latex bicouche amovible prélixivié dont la forme est revêtue ;
xii. tremper le film de latex bicouche amovible prélixivié obtenu à l'étape (xi) dans une solution polymérique pour produire un film de latex bicouche amovible revêtu de polymère ;
xiii. faire perler le film de latex bicouche amovible revêtu de polymère dont la forme est revêtue obtenu à l'étape (xii) pour produire un film de latex bicouche amovible perlé dont la forme est revêtue ;
xiv. faire durcir le film de latex bicouche amovible perlé dont la forme est revêtue obtenu à l'étape (xiii) pour produire un gant bicouche amovible durci dont la forme est revêtue ;
xv. post-lixivier le gant bicouche amovible durci dont la forme est revêtue obtenu à l'étape (xiv) pour produire un gant bicouche amovible post-lixivié dont la forme est revêtue ;
xvi. faire sécher le gant bicouche amovible post-lixivié dont la forme est revêtue obtenu à l'étape (xv) pour produire le gant bicouche amovible revêtu de polymère,
**caractérisé en ce que** la couche adhésive produite à l'étape (vii) comporte un agent mouillant, un agent de coagulation de latex, un polymère organique synthétique et de l'eau, dans lequel l'agent mouillant est utilisé en une quantité comprise entre 0,40 % et 0,80 % en poids de la couche adhésive, dans lequel l'agent de coagulation de latex est utilisé en une quantité comprise entre 17,00 % et 20,00 % en poids de la couche adhésive, dans lequel le polymère organique synthétique est utilisé en une quantité comprise entre 10,00 % et 14,00 % en poids de la couche adhésive et dans lequel de l'eau est utilisée en une quantité comprise entre 65,20 % et 72,60 % en poids de la couche adhésive.

16. Procédé de décollement d'un gant bicouche amovible selon les revendications 1 à 15, dans lequel le procédé comprend les étapes suivantes :
i. tirer sur la zone de poignet de la couche de gant extérieure jusqu'à ce que sa circonférence totale soit détachée de la couche de gant intérieure ; et
ii. décoller la couche de gant extérieure dès que la circonférence totale de la couche de gant extérieure a été détachée de la couche de gant intérieure pour parvenir à la couche de gant intérieure pour une autre utilisation.
